# EUROPEAN PATENT APPLICATION

(11) **EP 4 576 107 A1**
(43) Date of publication of application: **25.06.2025**
(21) Application number: 24222269.3
(22) Date of filing: 20.12.2024
(51) Int. Cl.: G16H 10/60, H04L 67/12

(54) **EFFICIENT TRANSFER OF PATIENT DATA**

(30) Priority: 21.12.2023 US 202363613685 P
(71) Applicant: Physio-Control, Inc., Redmond, WA 98052 (US)
(72) Inventor: FROLOV, Alexander, Redmond, 98052 (US); DUKE, Steven Barry, Redmond, 98052 (US); PADMANABHA, Seshadri K., Redmond, 98052 (US); RUTZER, Mark, Redmond, 98052 (US); STEWART, David B., Redmond, 98052 (US); DREW, Kevin, Redmond, 98052 (US); KILLEBREW, Mark G., Redmond, 98052 (US); SKELTON, Dennis M., Redmond, 98052 (US)
(74) Representative: V.O.

(57) **Abstract**

A method includes receiving patient data attributes of patient data collected by the medical device and an indication of a destination computer for the patient data. The method includes generating, based at least on data processing capabilities of the destination computer and the patient data attributes of the patient data, a set of transmission parameters for the medical device to adhere to when sending any of the patient data. The method includes sending, by the server, the set of transmission parameters to the medical device. The method includes receiving, by the server, at least a subset of the patient data from the medical device in response to sending the set of transmission parameters to the medical device. The subset of the patient data is in compliance with the set of transmission parameters. The method includes providing the subset of the patient data to the destination computer.

## Description

### Field

The present disclosure generally relates to patient medical data, and more particularly, to efficiently transferring patient medical data from a medical device to a destination computer.

### Background

Paramedics can use different medical devices to treat patients and collect patient data. As a non-limiting example, upon arriving at an emergency scene to treat a particular patient, paramedics can use a defibrillator to administer a controlled electric shock proximate to the heart of the particular patient to stop the fibrillation of the heart. Along with other medical devices, the defibrillator can collect patient data associated with the particular patient. For example, the defibrillator, along with other patient monitoring devices, can collect information indicating the particular patient's pulse, the particular patient's oxygen levels, etc.
While transporting the particular patient to a hospital, the paramedics can use the medical devices (e.g., the defibrillator and the other patient monitoring devices) to send the collected patient data to the hospital. However, sending the collected patient data to the hospital may be a time-intensive process and, in some scenarios, the hospital may not be able to receive (or process) all of the collected patient data.

### Summary

In one aspect, the present application discloses a server. The server includes a memory and a processor coupled to the memory. The processor is configured to receive, from a medical device, patient data attributes of patient data collected by the medical device and an indication of a destination computer for the patient data. The processor is also configured to generate, based at least on data processing capabilities of the destination computer and the patient data attributes of the patient data, a set of transmission parameters for the medical device to adhere to when sending any of the patient data. The processor is also configured to send the set of transmission parameters to the medical device. The processor is also configured to receive at least a subset of the patient data from the medical device in response to sending the set of transmission parameters to the medical device. The subset of the patient data is in compliance with the set of transmission parameters. The processor is also configured to provide the subset of the patient data to the destination computer.

In another aspect, the present application discloses a method. The method includes receiving, by a server and from a medical device, patient data attributes of patient data collected by the medical device and an indication of a destination computer for the patient data. The method also includes generating, based at least on data processing capabilities of the destination computer and the patient data attributes of the patient data, a set of transmission parameters for the medical device to adhere to when sending any of the patient data. The method also includes sending, by the server, the set of transmission parameters to the medical device. The method also includes receiving, by the server, at least a subset of the patient data from the medical device in response to sending the set of transmission parameters to the medical device. The subset of the patient data is in compliance with the set of transmission parameters. The method also includes providing, by the server, the subset of the patient data to the destination computer.

In another aspect, the present application discloses a non-transitory computer-readable medium. The non-transitory computer-readable medium includes instructions that, when executed by a processor within a server, causes the processor to perform operations. The operations include receiving, from a medical device, patient data attributes of patient data collected by the medical device and an indication of a destination computer for the patient data. The operations also include generating, based at least on data processing capabilities of the destination computer and the patient data attributes of the patient data, a set of transmission parameters for the medical device to adhere to when sending any of the patient data. The operations also include sending the set of transmission parameters to the medical device and receiving at least a subset of the patient data from the medical device in response to sending the set of transmission parameters to the medical device. The subset of the patient data is in compliance with the set of transmission parameters. The method also includes providing the subset of the patient data to the destination computer.

The foregoing summary is illustrative only and is not intended to be in any way limiting. In addition to the illustrative aspects, embodiments, and features described above, further aspects, embodiments, and features will become apparent by reference to the figures and the following detailed description.

### Brief Description of the Drawings

A more complete understanding of embodiments of the present application may be derived by referring to the detailed description and claims when considered in conjunction with the following figures, wherein like reference numbers may refer to similar elements throughout the figures. The figures are provided to facilitate understanding of the disclosure without limiting the breadth, scope, scale, or applicability of the disclosure. The drawings are not necessarily made to scale.
Figure 1 illustrates a system that is operable to efficiently transfer patient data from a medical device to a destination computer, according to an exemplary embodiment;
Figure 2 illustrates a negotiation protocol for efficiently transferring patient data from a medical device to a destination computer, according to an exemplary embodiment;
Figure 3 illustrates a process for selecting a communications means for uploading patient data to a server, according to an exemplary embodiment;
Figure 4 illustrates a process for determining whether to add particular patient data to a set of transmission parameters, according to an exemplary embodiment; and
Figure 5 is a flowchart of a method for efficiently transferring patient data from a medical device to a destination computer, according to an exemplary embodiment.

### Detailed Description

The following description describes techniques for efficiently transferring patient medical data. A medical device can be configured to collect patient data and transmit the collected patient data to a third party. As a non-limiting example, paramedics can use the medical device (e.g., a defibrillator, a patient monitoring device, etc.) to (i) collect patient data associated with a patient and (ii) wirelessly send the collected patient data to a destination computer (e.g., a destination hospital, an urgent care center, a device associated with a destination hospital or urgent care center, etc.). By sending the patient data to the destination computer while the paramedics are in route to the destination hospital, the destination hospital can be better prepared to treat the patient upon arrival.

In some scenarios, the medical device can upload the patient data to a server. After receiving the patient data, the server can provide the patient data to the destination computer. However, sending (e.g., uploading) the patient data to the server can be a time-intensive process. For example, the time required to upload the patient data to the server can vary based on the size of the patient data, the contents of the patient data, the communications means used to upload the patient data (e.g., Institute of Electrical and Electronics Engineers (IEEE) 802.11 (WiFi), cellular, hotspot, etc.), and other factors.

Furthermore, in some scenarios, the destination computer may not be able to process all of the patient data from the server or may only be able to process a subset of the patient data from the server. For example, in some scenarios, the destination computer may be temporarily unavailable (e.g., "offline") or may have limited resources (e.g., limited data storage availability, limited software to access the patient data, etc.). In these scenarios, after the medical device takes a relatively long time to upload the patient data to the server, the patient data (or a substantial portion of the patient data) may not be processed by the destination computer. Additionally, depending on the communications means used to upload the patient data, the cost of uploading the patient data to the server can be relatively expensive, while the objective of providing the patient data to the destination computer for processing remains unfulfilled or partially unfulfilled. For example, using cellular communications to upload the patient data to the server can be significantly more expensive than using WiFi communications to upload the patient data to the server.

To ensure that patient data uploaded to the server (by the medical device) is successfully provided to the destination computer in such a manner that the destination computer can process the patient data, a negotiated data transfer between the medical device and the server occurs. To illustrate, prior to uploading the patient data to the server, the medical device can provide a variety of different attributes associated with the patient data to the server. As non-limiting examples, the attributes can indicate formats (e.g., data types) of patient data to be uploaded to the server, a data size of the patient data to be uploaded to the server, an intended destination (e.g., the destination computer) for the patient data to be uploaded to the server, an urgency metric for delivering some or all of the patient data to the intended destination, a priority metric associated with some or all of the patient data, a measured quality of service (QoS) parameters associated with transmitting the patient data, an amount of certain data to be uploaded to the server, a time period for sending some of the patient data, a fidelity metric associated with sending at least some of the patent data, available communication mediums to transmit the patient data, etc.

In response to receiving the attributes from the medical device, the server can determine or identify data processing capabilities of the intended destination (e.g., the destination computer) and compare the data processing capabilities of the intended destination with the attributes from the medical device. In particular, the server can determine an availability of the destination computer to receive the patient data, an amount of available storage at the destination computer, different data formats that are able to be processed by the destination computer, different resources that are available at the destination computer, whether there is an indication that the destination computer is awaiting the patient data, etc. In some scenarios, the destination computer can inform the server of its data processing capabilities in response to a query from the server. In other scenarios, the server can store the data processing capabilities of the destination computer in a database accessible to the server. In these scenarios, the database can be periodically updated to ensure that the server has the most current data processing capabilities of the destination computer. Additionally, the server can determine how the medical device can efficiently upload the patient data. For example, the server can determine whether the medical device should use WiFi communications to upload the patient data, cellular communications to upload the patient data, etc. The determination can be based on an urgency level for providing the patient data to the destination computer and the measured QoS parameters associated with uploading the patient data to the server.

Based on the above-mentioned determinations, the server can generate a set of transmission parameters for the medical device. Among other things, the set of transmission parameters can indicate (i) whether all of the collected patient data should be uploaded to the server, (ii) whether a subset of the patient data should be uploaded to the server, (iii) a communications means for uploading all (or a subset) of the patient data, etc. In some embodiments, the set of transmission parameters can also indicate (i) whether the destination computer is available to receive the patient data, (ii) data formats for patient data based on the resources available at the destination computer, (iii) how much patient data (e.g., a data size of the patient data) the medical device should upload, (iv) an expected duration for the transmission (e.g., how long before the destination computer receives the patient data), etc. As a non-limiting example, if the server determines that the destination computer has resources to playback audio but does not have resources to playback video, the set of transmission parameters can indicate the medical device should bypass uploading the patient data that includes video files. Thus, the server can recommend that a subset of the patient data is sent, as the excluded patient data (e.g., the patient data not in the subset) would not be usable at the destination computer and would increase the transmission time.

The server can send the set of transmission parameters to the medical device and, upon receiving the set of transmission parameters, the medical device can upload (e.g., send) the patient data that is recommended by the set of transmission parameters using the communications means recommended by the set of transmission parameters. After the medical device uploads the recommended patient data to the server, the server can send the patient data to destination computer.

Thus, the techniques described herein ensure that the patient data uploaded to the server (by the medical device) is successfully provided to the intended destination in such a manner that the patient data can be processed. For example, by determining the data processing capabilities of the intended destination and negotiating the data transfer between the medical device and the server based on those data processing capabilities, the server can recommend, to the medical device, which portion of the patient data will be usable by the intended destination and which portion of the patient data will be unusable by the intended destination. As a result, the medical device can bypass uploading patient data that will not be accepted by the intended destination. Bypassing the uploading of patient data that will not be accepted reduces the amount of patient data uploaded to the server, decreases transmission times (e.g., the time it takes to upload), and reduces costs, thus improving the efficiency of the communication transmission itself, especially if the patient data is uploaded using cellular communications. Such improved efficiencies are particularly important in the context of medical device transmissions, where uploading of unnecessary or unusable data could result in delays in responding to a medical event, identifying life-threating indications and/or treating patients.

The figures and the following description illustrate specific exemplary embodiments. It will be appreciated that those skilled in the art will be able to devise various arrangements that, although not explicitly described or shown herein, embody the principles described herein and are included within the scope of the claims that follow this description. Furthermore, any examples described herein are intended to aid in understanding the principles of the disclosure and are to be construed as being without limitation. As a result, this disclosure is not limited to the specific embodiments or examples described below, but by the claims and their equivalents.

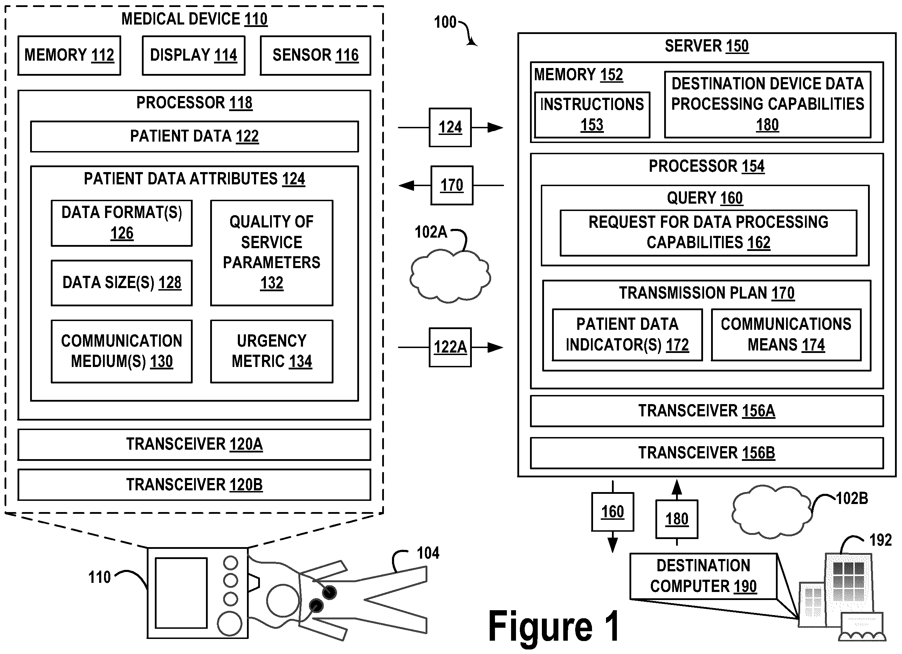

Particular implementations are described herein with reference to the drawings. In the description, common features may be designated by common reference numbers throughout the drawings. In some drawings, multiple instances of a particular type of feature are used. Although these features are physically and/or logically distinct, the same reference number is used for each, and the different instances are distinguished by addition of a letter to the reference number. When the features as a group or a type are referred to herein (e.g., when no particular one of the features is being referenced), the reference number is used without a distinguishing letter. However, when one particular feature of multiple features of the same type is referred to herein, the reference number is used with the distinguishing letter. For example, referring to Figure 1, networks are illustrated and associated with reference number 102. When referring to a particular one of the networks, such as the network 102A, the distinguishing letter "A" is used. However, when referring to any arbitrary one of the networks or to the networks as a group, the reference number 102 may be used without a distinguishing letter.

As used herein, various terminology is used for the purpose of describing particular implementations only and is not intended to be limiting. For example, the singular forms "a," "an," and "the" are intended to include the plural forms as well, unless the context clearly indicates otherwise. Further, the terms "comprise," "comprises," and "comprising" are used interchangeably with "include," "includes," or "including." Additionally, the term "wherein" is used interchangeably with the term "where." As used herein, "exemplary" indicates an example, an implementation, and/or an aspect, and should not be construed as limiting or as indicating a preference or a preferred implementation. As used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). As used herein, the term "set" refers to a grouping of one or more elements, and the term "plurality" refers to multiple elements.

Referring to Figure 1, a system 100 that is operable to efficiently transfer patient data from a medical device to a destination computer is illustrated, in accordance with an exemplary embodiment. The system 100 includes a medical device 110, a server 150, and a destination computer 190. As illustrated in Figure 1, in some implementations, the destination computer 190 can be located inside of a medical care facility 192, such as a hospital or an urgent care center.

The medical device 110 can communicate with the server 150 using a network 102A. For example, the medical device 110 can send data to the server 150 using the network 102A, and the server 150 can send data to the medical device 110 using the network 102A. According to one implementation, the network 102A is a WiFi network. According to one implementation, the network 102A is a cellular network, such as a public land mobile network (PLMN). The server 150 can communicate with the destination computer 190 using a network 102B. For example, the server 150 can send data to the destination computer 190 using the network 102B, and the destination computer 190 can send data to the server 150 using the network 102B. According to one implementation, the network 102B is a WiFi network. According to one implementation, the network 102B is a cellular network, such as a PLMN. In some embodiments, the networks 102A, 102B can correspond to the same network. In other embodiments, the networks 102A, 102B can be different. For example, in some scenarios, the network 102A can be a cellular network, and the network 102B can be a WiFi network. In other scenarios, the network 102B can be a cellular network, and the network 102A can be a WiFi network.

The medical device 110 can be used to treat a patient 104. As a non-limiting example, in Figure 1, the medical device 110 is depicted as a defibrillator that is operable to perform defibrillation operations on the patient 104. It should be understood that the defibrillator depicted in Figure 1 is merely one non-limiting example of the medical device 110. In other implementations, the medical device 110 can include a patient monitoring device, a ventilator, a drug administration pump. or any other device that is operable to treat or monitor the patient 104.

The medical device 110 includes a processor 118, a memory 112 coupled to the processor 118, a display 114 coupled to the processor 118, one or more sensors 116 coupled to the processor 118, a transceiver 120A coupled to the processor 118, and a transceiver 120B coupled to the processor 118. The transceiver 120A can be operable to send and receive data using a first communication protocol. As a non-limiting example, the transceiver 120A can be tuned to send and receive data using a WiFi communications protocol. Thus, in the scenario where the network 102A corresponds to a WiFi network, the transceiver 120A can be used to send and receive data from the server 150 over the WiFi network 102A. The transceiver 120B can be operable to send and receive data using a second communication protocol. As a non-limiting example, the transceiver 120B can be tuned to send and receive data using a cellular communications protocol. Thus, in the scenario where the network 102A correspond to a cellular communications network (e.g., a PLMN), the transceiver 120B can be used to send and receive data from the server 150 over the cellular communications network 102A. In some embodiments, the medical device 110 can include a single transceiver 120 that is tunable to send and receive data using different protocols (e.g., tunable to communicate over different frequency bands).

The sensor 116 can be configured to sense (e.g., detect) the patient data 122 from the patient 104. The patient data 122 can include any medical data that can be obtained using a medical device, such as the medical device 110. Non-limiting examples of the patient data 122 can include glucose levels of the patient 104, heartrate activity of the patient 104, ECG patterns of the patient 104, oxygen levels of the patient 104, or any other metric associated with the health of the patient 104 that is measurable by a medical device, such as the medical device 110. In some scenarios, the patient data 122 can also include video, audio, or both. As a non-limiting example, the medical device 110 can be configured to capture video and/or audio of the patient 104. In some scenarios, the patient data 122 can include writings (e.g., charts, notes, etc.) generated by paramedics (or other healthcare workers) that treated the patient 104 at an accident scene. In general, the patient data 122 can include any information that is usable to determine a health status associated with the patient 104.

Paramedics can use the medical device 110 to (i) collect the patient data 122 of the patient 104 and (ii) wirelessly send the patient data 122 to the destination computer 190. By sending the patient data 122 to the destination computer 190 while the paramedics are in route to the medical care facility 192, the medical care facility 192 can be better prepared to treat the patient 104 upon arrival.

However, prior to initiating transmission of the patient data 122 to the destination computer 190, the processor 118 can be configured to generate patient data attributes 124 of the patient data 122 collected by the medical device 110. The patient data attributes 124 can include data formats 126 of the patient data 122, a data size 128 of the patient data 122 as whole (or data sizes 128 of different elements of the patient data 122), available communication mediums 130 for transmitting the patient data 122, quality of service parameters 132 associated with transmitting the patient data 122, an urgency metric 134 for delivering the patient data 122 to the destination computer 190, etc. In general, the patient data attributes 124 can include any descriptive information indicative of the patient data 122 or any information indicating transmission parameters (or options) for delivering the patient data 122. The medical device 110 can be configured to send the patient data attributes 124 and an indication of the destination computer 190 for the patient data 122 to the server 150 via the network 102A.

The server 150 includes a processor 154, a memory 152 coupled to the processor 154, a transceiver 156A coupled to the processor 154, and a transceiver 156B coupled to the processor 154. The memory 152 corresponds to a non-transitory computer-readable medium that stores instructions 153 that are executable by the processor 154 to perform the operations described herein.

The transceiver 156A can be operable to send and receive data using a first communication protocol. As a non-limiting example, the transceiver 156A can be tuned to send and receive data using a WiFi communications protocol. Thus, in the scenario where the network 102A (or the network 102B) corresponds to a WiFi network, the transceiver 156A can be used to send and receive data from the medical device 110 (or the destination computer 190) over the WiFi network 102A (or the WiFi network 102B). The transceiver 156B can be operable to send and receive data using a second communication protocol. As a non-limiting example, the transceiver 156B can be tuned to send and receive data using a cellular communications protocol. Thus, in the scenario where the network 102A (or the network 102B) correspond to a cellular communications network (e.g., a PLMN), the transceiver 156B can be used to send and receive data from the medical device 110 (or the destination computer 190) over the cellular communications network 102B (or the cellular communications network 102B). In some embodiments, the server 150 can include a single transceiver 156 that is tunable to send and receive data using different protocols (e.g., tunable to communicate over different frequency bands). The server 150 can be configured to receive, from the medical device 110, the patient data attributes 124 of the patient data 122 collected by the medical device 110 and an indication of the destination computer 190 for the patient data 122. For example, the transceiver 156 can receive the patient data attributes 124 from the medical device 110 via the network 102A.

To determine whether the medical device 110 should send (e.g., upload) all of the patient data 122 to the server 150 or a subset of the patient data 122 to the server 150, the server 150 can determine data processing capabilities 180 of the destination computer 190. In particular, the data processing capabilities 180 of the destination computer 190 can indicate whether the destination computer 190 is able to receive/process (i) all of the patient data 122 from the server 150 or (ii) only a subset of the patient data 122 from the server 150. For example, in some scenarios, the destination computer 190 may be temporarily unavailable (e.g., "offline") or may have a limited resources (e.g., data storage availability or software to access the patient data 122). In these scenarios, after the medical device 110 takes a relatively long time to upload the patient data 122 to the server 150, the patient data 122 (or a substantial portion of the patient data 122) may not be received/processed by the destination computer 190. The data processing capabilities 180 of the destination computer 190 can also be used to determine a communications means 174 for uploading the patient data 122 to the server 150. For example, depending on the communications means 174 used to upload the patient data 122, the cost of uploading the patient data 122 to the server 150 can be relatively expensive, while the objective of providing the patient data 122 to the destination computer 190 for processing remains unfulfilled or partially unfulfilled. For example, using cellular communications to upload the patient data 122 to the server 150 can be significantly more expensive than using WiFi communications to upload the patient data 122 to the server 150. The increase in cost may not be an adequate tradeoff if all of the patient data 122 is not delivered to the destination computer 190.

To determine the data processing capabilities 180 of the destination computer 190, the processor 154 of the server 150 can be configured to generate a query 160 that includes a request 162 for the data processing capabilities 180 of the destination computer 190. In some scenarios, the server 150 can be configured to send, via the transceiver 156 and the network 102B, the query 160 to the destination computer 190 in response to receiving the patient data attributes 124 (and the indication of the destination computer 190) from the medical device 110. In other scenarios, the server 150 can be configured to periodically send the query 160 to the destination computer 190 via the transceiver 156 and the network 102B. In response to receiving the query 160, the destination computer 190 can be configured to send an indication of the data processing capabilities 180 of the destination computer 190 to the server 150.

The server 150 can be configured to receive, from the destination computer 190, the indication of the data processing capabilities 180 of the destination computer 190 in response to sending the query 160. For example, the destination computer 190 can send the indication of the data processing capabilities 180 of the destination computer 190 via the network 102B. The data processing capabilities 180 of the destination computer 190 can indicate an availability of the destination computer 190 to receive the patient data 122, an amount of available storage at the destination computer 190, different data formats are able to be processed by the destination computer 190, etc.

The processor 154 of the server 150 can be configured to generate, based at least on the data processing capabilities 180 of the destination computer 190 and the patient data attributes 124 of the patient data 122, a set of transmission parameters 170 for the medical device 110 to adhere to when sending (e.g., uploading) any of the patient data 122. The set of transmission parameters 170 includes one or more patient data indicators 172 that indicate a subset of the patient data 122 for the medical device 110 to send (e.g., upload), and the set of transmission parameters 170 also includes the communications means 174 for sending the subset of the patient data 122. In some scenarios, the subset of the patient data 122 indicated by the one or more patient data indicators 172 can be determined based on a data format of particular patient data 122 and whether the data processing capabilities 180 indicate that the destination computer 190 can process data having the data format. For example, in response to a determination that a first format of data is not compatible with the data processing capabilities 180 of the destination computer 190, the set of transmission parameters 170 indicates to the medical device 110 to bypass transmission of the patient data 122 having the first format.

In some scenarios, the communications means 174 for sending the subset of the patient data 122 corresponds to a cellular communications means or a WiFi communications means. The communications means 174 can be determined by the server 150 based at least in part on some of the patient data attributes 124. To illustrate, the communications means 174 can be determined at least in part on the urgency metric 134 associated with the patient data 122, the quality of service parameters 132 associated with sending the patient data 122 to the server 150, the available communication mediums 130 for sending the patient data 122 to the server 150, or the data size(s) 128 of the patient data 122. As a non-limiting example, if the urgency metric 134 indicates that it is urgent the destination computer 190 receive the patient data 122 and the quality of service parameters 132 indicate that the WiFi network 102A is congested, the communication means 174 can indicate to the medical device 110 to send the patient data 122 using cellular communications.

The server 150 can be configured to send the set of transmission parameters 170 to the medical device 110. For example, the server 150 can send the set of transmission parameters 170 to the medical device 110 via the network 102A. In response to receiving the set of transmission parameters 170, the medical device 110 can send at least a subset 122A of the patient data 122 (or all of the patient data 122) to the server 150 while adhering to the set of transmission parameters 170. For example, the medical device 110 can send the subset 122A of the patient data 122 to the server 150, via the network 102A, using the communications medium 174 indicated in the set of transmission parameters 170.

The server 150 can be configured to receive at least the subset 122A of the patient data 122 (or all of the patient data 122) from the medical device 110 in response to sending the set of transmission parameters 170 to the medical device 110. As indicated above, the subset 122A of the patient data 122 is in compliance with the set of transmission parameters 170. In response to receiving at least the subset 122A of the patient data 122, the server 150 can be configured to provide the subset 122A of the patient data 122 to the destination computer 190. For example, the server 150 can send the subset 122A of the patient data 122 to the destination computer 190 via the network 102B.

The techniques described with respect to Figure 1 ensure that the patient data 122 uploaded to the server 150 by the medical device 110 is successfully provided to the destination computer 190 in such a manner that enables the destination computer 190 to process the patient data 122. For example, by determining the data processing capabilities 180 of the destination computer 10 and negotiating the data transfer between the medical device 110 and the server 150 based on those data processing capabilities 180, the server 150 can recommend, to the medical device 110, which portion of the patient data 122 will be usable by the destination computer 190 and which portion of the patient data 122 will be unusable by the destination computer 190. As a result, the medical device 110 can bypass uploading patient data 122 that will not be accepted by the destination computer 190. Bypassing the uploading of patient data 122 that will not be accepted reduces the amount of patient data 122 uploaded to the server 150, decreases transmission times (e.g., the time it takes to upload), and reduces costs, especially if the patient data 122 is uploaded using cellular communications.

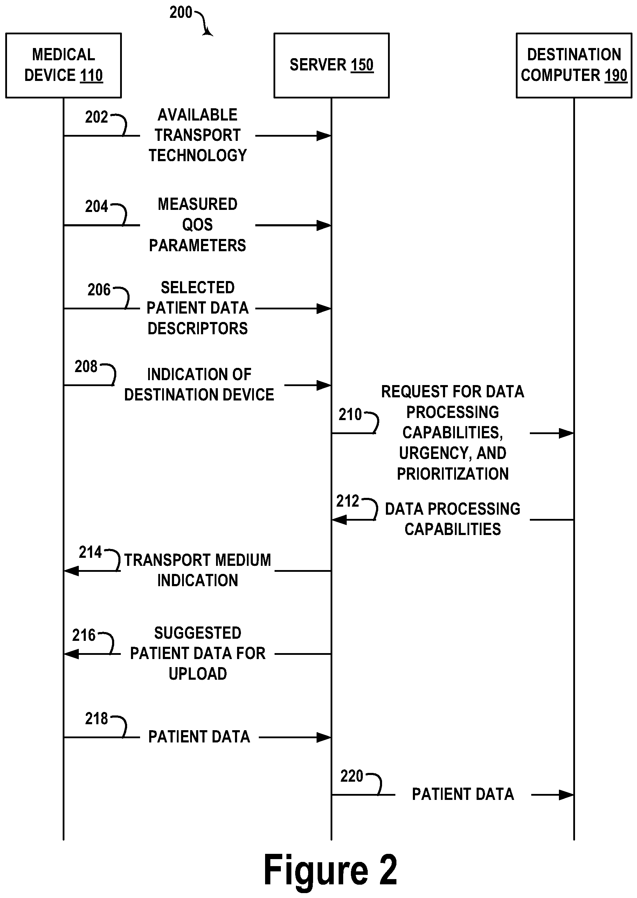

Referring to Figure 2, a negotiation protocol 200 for efficiently transferring patient data from a medical device to a destination computer is illustrated, in accordance with an exemplary embodiment. The negotiation protocol 200 includes an exchange of information between a medical device (e.g., the medical device 110), a server (e.g., the server 150), and a destination computer (e.g., the destination computer 190). It should be understood that the steps illustrated in the negotiation protocol 200 for illustrated purposes only and should not be construed as limiting. In some embodiments, one or more of the steps can be combined into a single step. In some embodiments, one or more of the steps can be omitted.

At step 202, the medical device 110 sends an indication of available transport technology to the server 150. The indication of available transport technology can indicate different communication means or mediums that can be used by the medical device 110 to send (e.g., upload) patient data to the server 150.

At step 204, the medical device 110 sends measured quality of service parameters to the server 150. For example, for each communication medium available to the medical device 110, the medical device 110 can indicate different quality of service parameters, such as packet loss, jitter, latency, bandwidth, etc. The quality of service parameters can be sent to the server 150 to enable the server 150 to determine which communication medium the medical device 110 should use when uploading the patient data 122 to the server 150.

At step 206, the medical device 110 sends selected patient data descriptors to the server 150. The selected patient data descriptors describe user-selected patient data 122 that a user of the medical device 110, such as a paramedic, intends to send to the destination computer 190. In particular, the selected patient data descriptors can describe formats of the user-selected patient data 122, data sizes of the user-selected patient data 122, an urgency metric associated with the user-selected patient data 122, etc. In some embodiments, the available transport technology, the measured quality of service parameters, and the selected patient data descriptors sent to the server 150 in steps 202-206 are included in the patient data attributes 124 described with respect to Figure 1.

At step 208, the medical device 110 sends an indication of the destination computer 190 to the server 150. In some embodiments, the information sent to the server 150 in steps 202-208 can be sent in a single transmission.

At step 210, the server 150 sends a request 162 for (1) data processing capabilities 180 of the destination computer 190, (2) an urgency indication for some or all of the patient data 122, and (3) a prioritization indication for some or all of the paitent data 122 to the destination computer 190. With regards to the prioritization indications, some patient data 122 can be more important than other patient data 122. For example, a 12-lead ECG report may be more important (e.g., a higher priority) than continuous vitals. At step 212, the destination computer 190 sends the data processing capabilities 180 of the destination computer 190 to the server 150. In some embodiments, the data processing capabilities 180 of the destination computer 190 are periodically provided to the server 150. Thus, in these embodiments, the server 150 does not have to request the data processing capabilities 180 of the destination computer 190.

At step 214, the server 150 sends a transport medium indication to the medical device 110. The transport medium indication indicates whether the medical device 110 should send patient data 122 using an IEEE 802.11 protocol, a cellular communication protocol, a hotspot, etc. The server 150 can determine the transport medium based on the measured quality of service parameters, the urgency metric associated with the user-selected patient data 122, costs, etc.

At step 216, the server 150 sends suggestions of patient data 122 for the medical device 110 to upload based on the data processing capabilities 180 of the destination computer 190. For example, the server 150 can identify portions of the patient data 122 that the destination computer 190 is not able to process and portions of the patient data 122 that the destination computer 190 is able to process. The suggestions from the server 150 can include the portions of the patient data 122 that the destination computer 190 is able to process. In some embodiments, the transport medium indication and the suggestions of patient data 122 for the medical device to upload in steps 214-216 are included in the set of transmission parameters 170 described with respect to Figure 1.

At step 218, the medical device 110 sends the suggested patient data 122 to the server 150 using the transport medium suggested by the server 150. At step 220, the server 150 sends the patient data 122 to the destination computer 190.

The negotiation protocol 200 described with respect to Figure 2 ensures that the patient data 122 uploaded to the server 150 by the medical device 110 is successfully provided to the destination computer 190 in such a manner that enables the destination computer 190 to process the patient data 122. For example, by determining the data processing capabilities 180 of the destination computer 10 and negotiating the data transfer between the medical device 110 and the server 150 based on those data processing capabilities 180, the server 150 can recommend, to the medical device 110, which portion of the patient data 122 will be usable by the destination computer 190 and which portion of the patient data 122 will be unusable by the destination computer 190. As a result, the medical device 110 can bypass uploading patient data 122 that will not be accepted by or processed by the destination computer 190. Bypassing the uploading of patient data 122 that will not be accepted reduces the amount of patient data 122 uploaded to the server 150, decreases transmission times (e.g., the time it takes to upload), and reduces costs, especially if the patient data 122 is uploaded using cellular communications.

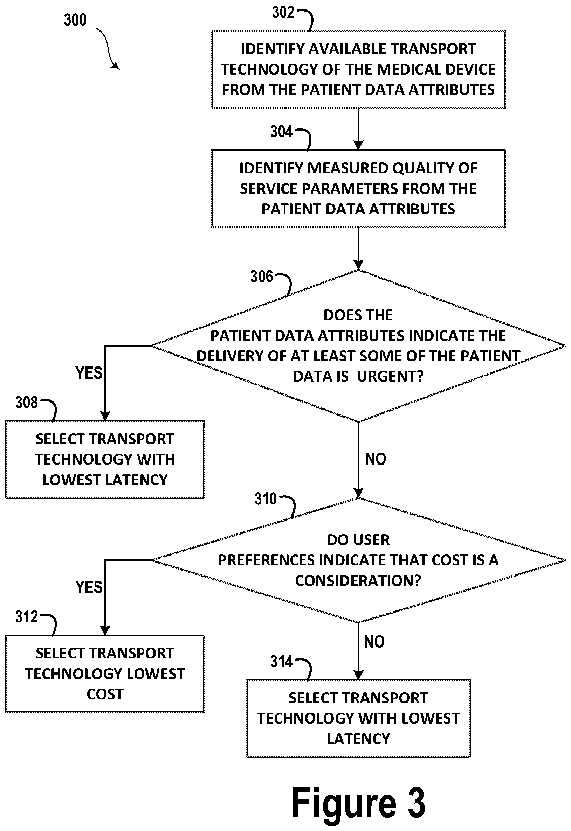

Figure 3 illustrates a process 300 for selecting a communications means for uploading patient data to a server, according to an exemplary embodiment. The process 300 can be performed by the server 150 of Figure 1.

According to the process 300, at step 302, the server 150 can identify available transport technologies of the medical device 110 from the patient data attributes 124. To illustrate, the patient data attributes 124 can indicate the available communication mediums 130 to the medical device 110. As non-limiting examples, the patient data attributes 124 can indicate that the medical device 110 can send and receive data using an IEEE 802.11 protocol, a cellular communication protocol, a hotspot, etc.

At step 304, the server 150 can identify measured quality of service parameters from the patient data attributes 124. For example, for available transport technology of the medical device 110, the patient data attributes 124 can indicate different quality of service parameters, such as packet loss, jitter, latency, bandwidth, etc.

At step 306, the server 150 can determine, based on the patient data attributes 124, whether delivery of at least some of the patient data 122 to the destination computer 190 is urgent. If delivery of the patient data 122 is urgent, at step 308, the server 150 can select the transport technology with the lowest latency. As a non-limiting example, if the quality of service parameters indicate that the cellular communication protocol has the lowest latency at that moment in time, the server 150 can indicate to the medical device 110 (in the set of transmission parameters 170) to upload the patient data 122 using the cellular communication protocol.

If delivery of the patient data 122 is not urgent, at step 310, the server 150 can determine whether user preferences from the medical device 110 indicate that cost should be a consideration. If the user preferences indicate that cost should be a consideration, at step 312, the server 150 can select the transport technology with the lowest cost. As a non-limiting example, if the using the IEEE 802.11 protocol is the cheapest way to upload the patient data 122, the server 150 can indicate to the medical device 110 (in the set of transmission parameters 170) to upload the patient data 122 using the IEEE 802.11 protocol. However, if the user preferences indicate that cost should not be a consideration, at step 314, the server 150 can select the transport technology with the lowest latency.

Although latency is used as the quality of service parameter in Figure 3, it should be understood that the techniques described herein can use different quality of service parameters (or a combination of quality of service parameters) to select the transport technology. For example, in some scenarios, the server 150 can apply different weights to each quality of service parameter to determine which transport technology provides the best overall means of communication.

The process 300 of Figure 3 enables the server 150 to select the transport technology (e.g., the communication means) for the medical device 110 to use when uploading the patient data 122 to the server 150 based quality of service parameters and user preferences.

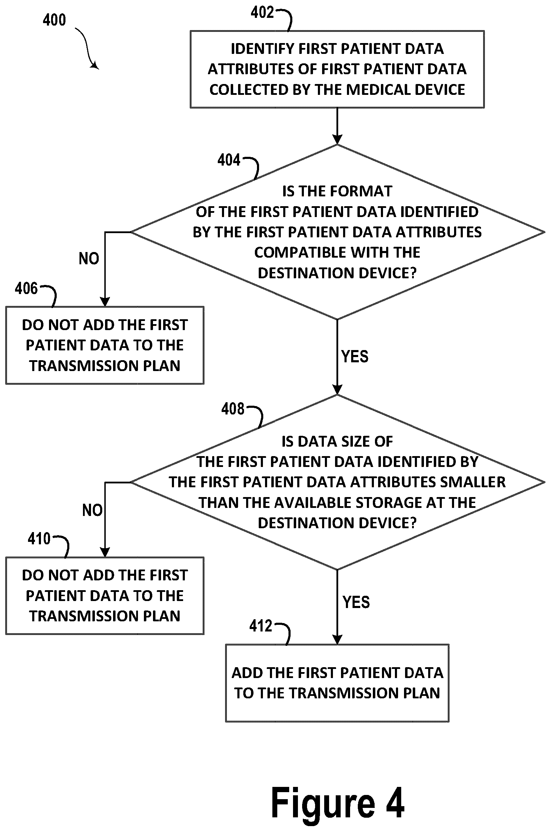

Figure 4 illustrates a process 400 for determining whether to add particular patient data to a set of transmission parameters, according to an exemplary embodiment. The process 400 can be performed by the server 150 of Figure 1.

According to the process 400, at step 402, the server 150 can identify first patient data attributes 124 of first patient data 122 collected by the medical device 110. The first patient data 122 can be a subset (or an item) of the cumulative patient data 122 collected by the medical device 110. As a non-limiting example, in one embodiment, the first patient data 122 is video captured by paramedics using the medical device 110. In this embodiment, the first patient data attributes 124 can indicate a format of the video (e.g., AVI, MP4, MOV, etc.), a data size of the video, etc. As another non-limiting example, in one embodiment, the first patient data 122 includes ECG patterns collected by the medical device 110. In this embodiment, the first patient data attributes 124 can indicate a format of the file that includes the ECG patterns, a data size of the file, etc. For ease of explanation, the process 400 depicted in Figure 4 describes the first patient data 122 as video captured by paramedics using the medical device 110. However, it should be understood that a similar process is used for patient data attributes of other patient data 122 collected by the medical device 110.

At step 404, the server 150 can determine whether the format of the first patient data 122 identified by the first patient data attributes 124 is compatible with the destination computer 190. As a non-limiting example, the data processing capabilities 180 of the destination computer 190 can indicate different formats of video the destination computer 190 can render and playback. The server 150 can identify the format of the video collected by the medical device 110 and determine whether the format is compatible with the data processing capabilities 180 of the destination computer 190. If the format of the video is not compatible with the data processing capabilities 180 of the destination computer 190, at step 406, the server 150 can bypass adding the first patient data 122 to the set of transmission parameters 170 for the medical device 110.

If the format of the video is compatible with the data processing capabilities 180 of the destination computer 190, at step 408, the server 150 can determine whether the data size of the first patient data 122 identified by the first patient data attributes 124 is smaller than the available storage at the destination computer 190. As a non-limiting example, the data processing capabilities 180 of the destination computer 190 can indicate an amount of available storage at the destination computer 190. The server 150 can determine whether data size of the video is less than the amount of available storage at the destination computer 190. If the data size of the video is not less than the amount of available storage at the destination computer 190, at step 410, the server 150 can bypass adding the first patient data 122 to the set of transmission parameters 170 for the medical device 110. However, if the data size of the video is less than the amount of available storage at the destination computer 190, at step 412, the server 150 can add the first patient data 122 to the set of transmission parameters 170 for the medical device 110.

The process 400 of Figure 4 ensures that the patient data 122 uploaded to the server 150 by the medical device 110 is compatible with the destination computer 190. For example, by determining the data processing capabilities 180 of the destination computer 10 and negotiating the data transfer between the medical device 110 and the server 150 based on those data processing capabilities 180, the server 150 can identify which portion of the patient data 122 is compatible with the destination computer 190 and which portion of the patient data 122 is not compatible with the destination computer 190. As a result, the medical device 110 can bypass uploading patient data 122 that is not compatible with the destination computer 190. Bypassing the uploading of patient data 122 that is not compatible with the destination computer 190 reduces the amount of patient data 122 uploaded to the server 150, decreases transmission times (e.g., the time it takes to upload), and reduces costs, especially if the patient data 122 is uploaded using cellular communications.

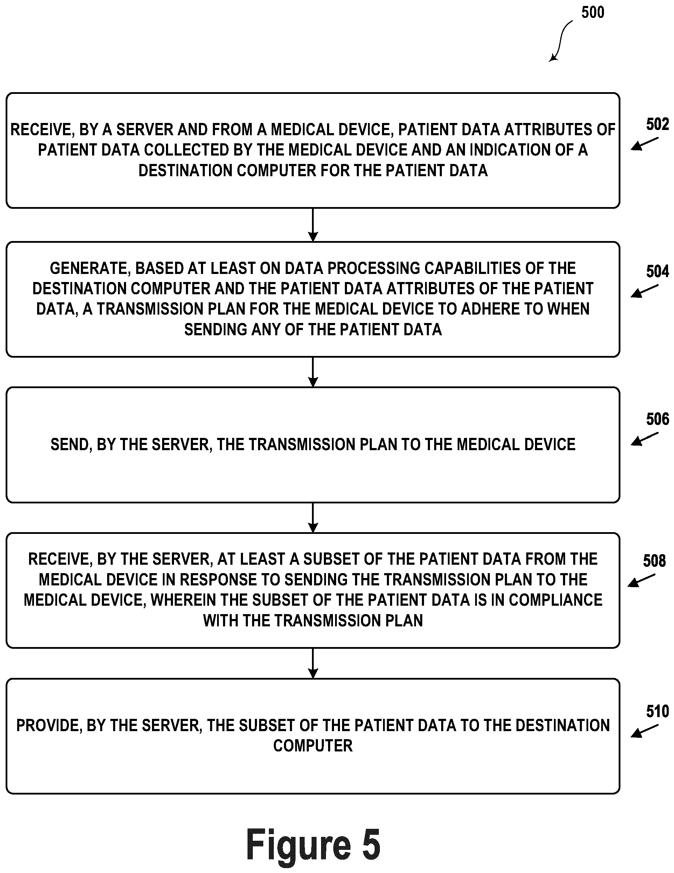

Figure 5 illustrates a flow chart of a method 500 for efficiently transferring patient data from a medical device to a destination computer, according to an exemplary embodiment. The method 500 can be performed by the server 150 of Figure 1.

The method 500 includes receiving, by a server and from a medical device, patient data attributes of patient data collected by the medical device and an indication of a destination computer for the patient data, at block 502. For example, referring to Figure 1, the server 150 receives, from the medical device 110, the patient data attributes 124 of the patient data 122 collected by the medical device 110 and the indication of the destination computer 190 for the patient data 122.

The method 500 also includes generating, based at least on data processing capabilities of the destination computer and the patient data attributes of the patient data, a set of transmission parameters for the medical device to adhere to when sending any of the patient data, at block 504. For example, referring to Figure 1, the server 150 generates, based at least on the data processing capabilities 180 of the destination computer 190 and the patient data attributes 124 of the patient data 122, the set of transmission parameters 170 for the medical device 110 to adhere to when sending any of the patient data 122.

The method 500 also includes sending, by the server, the set of transmission parameters to the medical device, at block 506. For example, referring to Figure 1, the server 150 sends the set of transmission parameters 170 to the medical device 110.

The method 500 also includes receiving, by the server, at least a subset of the patient data from the medical device in response to sending the set of transmission parameters to the medical device, at block 508. The subset of the patient data is in compliance with the set of transmission parameters. For example, referring to Figure 1, the server 150 receives at least a subset 122A of the patient data 122 from the medical device 110 in response to sending the set of transmission parameters 170 to the medical device 110. The subset 122A of the patient data 122 is in compliance with the set of transmission parameters 170.

The method 500 also include providing, by the server, the subset of the patient data to the destination computer, at block 510. For example, referring to Figure 1, the server 150 provides the subset 122A of the patient data 122 to the destination computer 190.

According to one implementation of the method 500, the patient data attributes 124 comprise at least one of data formats 126 of the patient data 122, a data size 128 of the patient data 122, quality of service parameters 132 associated with sending the patient data 122 to the server 150, available communication mediums 130 for sending the patient data 122 to the server 150, a prioritization metric associated with at least some of the patient data 122, a fidelity metric for sending at least some of the patient data 122, or an urgency metric 134 for delivering the patient data 122 to the destination computer 190.

According to one implementation of the method 500, the set of transmission parameters 170 indicates the subset 122A of the patient data 122 and a communication means 174 for sending the subset 122A of the patient data 122. The communications means 174 for sending the subset 122A of the patient data 122 corresponds to a cellular communication means or an IEEE 802.11 communications means.

According to one implementation of the method 500, in response to a determination that a first format of data is not compatible with the data processing capabilities 180 of the destination computer 190, the set of transmission parameters 170 indicates to the medical device 110 to bypass transmission of patient data 122 having the first format.

According to one implementation, the method 500 includes sending, by the server 150, a query 160 to the destination computer 190 in response to receiving the patient data attributes 124 and the indication of the destination computer 190 from the medical device 110. The query 160 includes a request 162 for the data processing capabilities 180 of the destination computer 190. The method 500 can also include receiving, by the server 150, an indication of the data processing capabilities 180 of the destination computer 190 in response sending the query 160.

According to one implementation, the method 500 includes periodically sending, by the server 150, a query 160 to destination computer 190. The query 160 includes a request 162 for the data processing capabilities 180 of the destination computer 190. The method 500 can also include receiving, by the server 150, an indication of the data processing capabilities 180 of the destination computer 190 in response sending each query 160.

According to one implementation of the method 500, the data processing capabilities 180 of the destination computer 190 indicate an availability of the destination computer 190 to receive the patient data 122, an amount of available storage at the destination computer 190, and different formats of data that are able to be processed by the destination computer 190.

The method 500 of Figure 5 ensures that the patient data 122 uploaded to the server 150 by the medical device 110 is successfully provided to the destination computer 190 in such a manner that enables the destination computer 190 to process the patient data 122. For example, by determining the data processing capabilities 180 of the destination computer 10 and negotiating the data transfer between the medical device 110 and the server 150 based on those data processing capabilities 180, the server 150 can recommend, to the medical device 110, which portion of the patient data 122 will be usable by the destination computer 190 and which portion of the patient data 122 will be unusable by the destination computer 190. As a result, the medical device 110 can bypass uploading patient data 122 that will not be accepted by the destination computer 190. Bypassing the uploading of patient data 122 that will not be accepted reduces the amount of patient data 122 uploaded to the server 150, decreases transmission times (e.g., the time it takes to upload), and reduces costs, especially if the patient data 122 is uploaded using cellular communications.

Additionally, instances in this specification where one element is "coupled" to another element can include direct and indirect coupling. Direct coupling can be defined as one element coupled to and in some contact with another element. Indirect coupling can be defined as coupling between two elements not in direct contact with each other, but having one or more additional elements between the coupled elements. Further, as used herein, securing one element to another element can include direct securing and indirect securing. Additionally, as used herein, "adjacent" does not necessarily denote contact. For example, one element can be adjacent another element without being in contact with that element.

As used herein, a system, apparatus, structure, article, element, component, or hardware "configured to" perform a specified function is indeed capable of performing the specified function without any alteration, rather than merely having potential to perform the specified function after further modification. In other words, the system, apparatus, structure, article, element, component, or hardware "configured to" perform a specified function is specifically selected, created, implemented, utilized, programmed, and/or designed for the purpose of performing the specified function. As used herein, "configured to" denotes existing characteristics of a system, apparatus, structure, article, element, component, or hardware which enable the system, apparatus, structure, article, element, component, or hardware to perform the specified function without further modification. For purposes of this disclosure, a system, apparatus, structure, article, element, component, or hardware described as being "configured to" perform a particular function may additionally or alternatively be described as being "adapted to" and/or as being "operative to" perform that function.

The flow chart diagrams included herein are generally set forth as logical flow chart diagrams. As such, the depicted order and labeled steps are indicative of one embodiment of the presented method. Other steps and methods may be conceived that are equivalent in function, logic, or effect to one or more steps, or portions thereof, of the illustrated method. Additionally, the format and symbols employed are provided to explain the logical steps of the method and are understood not to limit the scope of the method. Although various arrow types and line types may be employed in the flow chart diagrams, they are understood not to limit the scope of the corresponding method. Indeed, some arrows or other connectors may be used to indicate only the logical flow of the method. For instance, an arrow may indicate a waiting or monitoring period of unspecified duration between enumerated steps of the depicted method. Additionally, the order in which a particular method occurs may or may not strictly adhere to the order of the corresponding steps shown.

Unless otherwise indicated, the terms "first," "second," etc. are used herein merely as labels, and are not intended to impose ordinal, positional, or hierarchical requirements on the items to which these terms refer. Moreover, reference to, e.g., a "second" item does not require or preclude the existence of, e.g., a "first" or lower-numbered item, and/or, e.g., a "third" or higher-numbered item.

While the systems and methods of operation have been described with reference to certain examples, it will be understood by those skilled in the art that various changes can be made and equivalents can be substituted without departing from the scope of the claims. Therefore, it is intended that the present methods and systems not be limited to the particular examples disclosed, but that the disclosed methods and systems include all embodiments falling within the scope of the appended claims.

## Claims

1. A server comprising:
a memory; and
a processor coupled to the memory, the processor configured to:
receive, from a medical device, patient data attributes of patient data collected by the medical device and an indication of a destination computer for the patient data;
generate, based at least on data processing capabilities of the destination computer and the patient data attributes of the patient data, a set of transmission parameters for the medical device to adhere to when sending any of the patient data;
send the set of transmission parameters to the medical device;
receive at least a subset of the patient data from the medical device in response to sending the set of transmission parameters to the medical device, wherein the subset of the patient data is in compliance with the set of transmission parameters; and
provide the subset of the patient data to the destination computer.

2. The server of claim 1, wherein the patient data attributes comprise at least one of data formats of the patient data, a data size of the patient data, quality of service parameters associated with sending the patient data to the server, available communication mediums for sending the patient data to the server, a prioritization metric associated with at least some of the patient data, a fidelity metric for sending at least some of the patient data, a time range for sending at least some of the patient data, or an urgency metric for delivering the patient data to the destination computer.

3. The server of claim 1, wherein the set of transmission parameters indicates the subset of the patient data and a communications means for sending the subset of the patient data, wherein optionally
the communications means for sending the subset of the patient data corresponds to a cellular communications means or an Institute of Electrical and Electronics Engineers (IEEE) 802.11 communications means.

4. The server of claim 1, wherein, in response to a determination that a first format of data is not compatible with the data processing capabilities of the destination computer, the set of transmission parameters indicates to the medical device to bypass transmission of patient data having the first format.

5. The server of claim 1, wherein the processor is further configured to:
send a query to the destination computer in response to receiving the patient data attributes and the indication of the destination computer from the medical device, wherein the query includes a request for the data processing capabilities of the destination computer; and
receive an indication of the data processing capabilities of the destination computer in response sending the query.

6. The server of claim 1, wherein the processor is further configured to:
periodically send a query to destination computer, wherein the query includes a request for the data processing capabilities of the destination computer; and
receive an indication of the data processing capabilities of the destination computer in response sending each query.

7. The server of claim 1, wherein the data processing capabilities of the destination computer indicate an availability of the destination computer to receive the patient data, an amount of available storage at the destination computer, and different formats of data that are able to be processed by the destination computer, or
wherein the medical device comprises a defibrillator or a patient monitoring device.

8. A method comprising:
receiving, by a server and from a medical device, patient data attributes of patient data collected by the medical device and an indication of a destination computer for the patient data;
generating, based at least on data processing capabilities of the destination computer and the patient data attributes of the patient data, a set of transmission parameters for the medical device to adhere to when sending any of the patient data;
sending, by the server, the set of transmission parameters to the medical device;
receiving, by the server, at least a subset of the patient data from the medical device in response to sending the set of transmission parameters to the medical device, wherein the subset of the patient data is in compliance with the set of transmission parameters; and
providing, by the server, the subset of the patient data to the destination computer.

9. The method of claim 8, wherein the patient data attributes comprise at least one of data formats of the patient data, a data size of the patient data, quality of service parameters associated with sending the patient data to the server, available communication mediums for sending the patient data to the server, a prioritization metric associated with at least some of the patient data, a fidelity metric for sending at least some of the patient data, a time range for sending at least some of the patient data, or an urgency metric for delivering the patient data to the destination computer.

10. The method of claim 8, wherein the set of transmission parameters indicates the subset of the patient data and a communications means for sending the subset of the patient data,
wherein optionally the communications means for sending the subset of the patient data corresponds to a cellular communications means or an Institute of Electrical and Electronics Engineers (IEEE) 802.11 communications means.

11. The method of claim 8, wherein, in response to a determination that a first format of data is not compatible with the data processing capabilities of the destination computer, the set of transmission parameters indicates to the medical device to bypass transmission of patient data having the first format.

12. The method of claim 8, further comprising:
sending, by the server, a query to the destination computer in response to receiving the patient data attributes and the indication of the destination computer from the medical device, wherein the query includes a request for the data processing capabilities of the destination computer; and
receiving, by the server, an indication of the data processing capabilities of the destination computer in response sending the query.

13. The method of claim 8, further comprising:
periodically sending, by the server, a query to destination computer, wherein the query includes a request for the data processing capabilities of the destination computer; and
receiving, by the server, an indication of the data processing capabilities of the destination computer in response sending each query.

14. The method of claim 8, wherein the data processing capabilities of the destination computer indicate an availability of the destination computer to receive the patient data, an amount of available storage at the destination computer, and different formats of data that are able to be processed by the destination computer, or
wherein the medical device comprises a defibrillator or a patient monitoring device.

15. A non-transitory computer-readable medium comprising instructions that, when executed by a processor within a server, causes the processor to perform operations comprising:
receiving, from a medical device, patient data attributes of patient data collected by the medical device and an indication of a destination computer for the patient data;
generating, based at least on data processing capabilities of the destination computer and the patient data attributes of the patient data, a set of transmission parameters for the medical device to adhere to when sending any of the patient data;
sending the set of transmission parameters to the medical device;
receiving at least a subset of the patient data from the medical device in response to sending the set of transmission parameters to the medical device, wherein the subset of the patient data is in compliance with the set of transmission parameters; and
providing the subset of the patient data to the destination computer,
wherein optionally the medical device comprises a defibrillator or a patient monitoring device.
